# EUROPEAN PATENT SPECIFICATION

(11) **EP 0 765 155 B1**
(45) Date of publication and mention of the grant of the patent: **08.12.1999**
(21) Application number: 95922518.6
(22) Date of filing: 07.06.1995
(51) Int. Cl.: A61K 7/48, A61K 7/00

(54) **NON-IRRITANT AQUEOUS LIPOSOME DISPERSIONS CONTAINING ALPHA-HYDROXYCARBOXYLIC ACIDS, ALPHA-KETOCARBOXYLIC ACIDS AND/OR SALICYCLIC ACID IN THEIR SALT FORMS**
ALPHA-HYDROXYCARBONSAEUREN UND/ODER SALICYLSAEURE IM IHREN SALZFORM ENTHALTENDE NICHTREIZENDE WAESSRIGE LIPOSOMENDISPERSION
DISPERSIONS AQUEUSES NON IRRITANTES DE LIPOSOMES CONTENANT, SOUS FORME DE SELS, DES ACIDES ALPHA-HYDROXYCARBOXYLIQUES, ALPHA-CETOCARBOXYLIQUES ET/OU DE L'ACIDE SALICYLIQUE

(30) Priority: 15.06.1994 DE 4420727
(43) Date of publication of application: 02.04.1997
(73) Proprietor: Rovi GmbH Handelsgesellschaft für Rohstoffe, 36381 Schlüchtern (DE)
(72) Inventor: RÖDING, Joachim, D-65207 Wiesbaden (DE); TEICHMÜLLER, Ernst, E., D-36381 Schlüchtern (DE); SEIGFRIED, Bernd, G., D-67117 Limburgerhof (DE)
(74) Representative: Sternagel, Hans-Günther, Dr.
(86) International application number: EP9502169
(87) International publication number: WO9534279

(56) References cited:
- EP-A- 0 582 503
- DE-C- 4 005 711
- FR-A- 2 591 105

## Description

The present invention concerns non-irritant aqueous liposome dispersions of phospholipids for topical application, containing α-hydroxycarboxylic acids, α-ketocarboxylic acids and/or salicylic acid in their salt forms.

Diverse milk products and fruit ferments have been employed for centuries in traditional medicine and skin care, although at first it was not known which ingredients were responsible for their effects. Later, the α-hydroxycarboxylic acids or α-ketocarboxylic acids contained in these "beauty products" were found to have keratinolytic and skin stimulating properties. Salicylic acid shows similar effects. For these reasons, individual members of these substance classes have been employed for some time in both dermatology and cosmetics.

Recent investigations of the mechanism of action of the α-hydroxycarboxylic and α-ketocarboxylic acids have shown that their application to the skin corresponds to a gentle chemical peeling. A "softening" of the lipid material in the upper layers of the stratum corneum accelerates the sloughing off of the outermost horny scales. In addition, the acids stimulate metabolism in the dermis, and natural skin functions are reactivated. The endogenous collagen and elastin synthesis activities of the skin are stimulated. This, together with the accelerated scaling off of the outer skin layers, has a beneficial effect on the softness and elasticity of the skin. The skin becomes markedly softer and smoother. Clear improvements in greasy or unclean skin have also been observed through the use of α-hydroxycarboxylic and α-ketocarboxylic acids.

Investigations have also shown that the effectiveness of the the substances decreases with increasing pH. According to test results presented on 21st March 1994 at the Advanced Technology Conference in Barcelona, Spain, most of the α-hydroxycarboxylic and α-ketocarboxylic acids showed very little efficacy at pH greater than 6. Optimal efficacy was observed at markedly lower pH values.

However, the use of formulations of these acids or salicylic acid with a strongly acid pH leads to severe skin irritation. Thus while α-hydroxycarboxylic, α-ketocarboxylic acids and salicylic acid at low pH stimulate cell regeneration, they simultaneously cause irritation of the skin areas treated.

Highly concentrated liposome dispersions containing malic acid, citric acid, glycolic acid or lactic acid for use in the manufacture of liposomal formulations are commercially available (ROVI GmbH, Mutterstadt). These preparations all have a pH value below 4.6, so as to ensure the effectiveness of the α-hydroxycarboxylic acid constituents. However, this low pH represents a considerable irritative potential when the dispersions are applied.

EP-A-0 249 561, WO 94/12154, WO 93/15720, and WO 90/03781 describe various pharmaceutical compositions containing liposomes and a α-hydroxycarboxylic acid or salicylic acid for different applications.

EP-A-0 616 799 which is an earlier European patent application and does not belong to the state of the art discloses an aqueous cosmetic composition containing liposomes, un-neutralized free salicylic acid being included within the membrane lipid layer of the liposome, and an organic, water-soluble membrane-impermeant base.

The object of the present invention is thus to provide a non-irritant formulation which contains α-hydroxycarboxylic acids, α-ketocarboxylic acids and/or salicylic acid in an effective form.

This object is achieved by an aqueous liposomal dispersion of phospholipids comprising: (a) one or more α-hydroxycarboxylic acids, one or more α-ketocarboxylic acids, or salicylic acid or mixtures therefrom and (b) one or more alcohols, characterized in that said dispersion has a physiological pH value in the range from 5 to 7.5 and said acid(s) is (are) present nearly completely in salt form.

Liposomes are spherical structures (vesicles) with an envelope of one or more lipid double layers (bilayers). They are usually manufactured from phospholipids and can be loaded with various active substances, which then become enclosed within the liposomes. The liposomes of the present invention have a preferred particle size of between 70 nm and 300 nm (diameter).

One or more or a mixture of α-hydroxycarboxylic acids, α-ketocarboxylic acids and/or salicylic acid are enclosed in the vesicles of said aqueous liposome dispersion.

In a preferred embodiment of the invention, liposomes from soya phospholipids with a phosphatidyl choline proportion of at least 73% by weight are used; a phosphatidyl choline proportion of between 73 and 79% by weight is more preferred. Phospholipid fractions can be used containing, for example, a) 73 to 79% by weight phosphatidyl choline, 0 to 6% by weight lysophosphatidyl choline, approx. 8% by weight phosphatidic acid, approx. 4% by weight phosphatidyl ethanolamine and approx. 9% by weight other lipids, or b) 90 to 96% by weight phosphatidyl choline and 0 to 6% by weight lysophosphatidyl choline or c) at least 95% by weight phosphatidyl choline and at most 2% by weight lysophosphatidyl choline.

The fatty acid radicals of the soya phospholipids are mainly unsaturated fatty acids, linoleic acid being the major component. A typical fatty acid composition is: 61 to 71% linoleic acid, 3 to 7% linolenic acid, 6 to 13% oleic acid, 10 to 15% palmitic acid and 1.5 to 4% stearic acid.

Alternatively, hydrogenated phospholipids with a phosphatidyl choline proportion of at least 73% by weight and preferably 75 to 95% by weight can be used.

In principle, liposomes can be loaded with any dermatologically active α-hydroxycarboxylic acid, α-ketocarboxylic acid or salicylic acid. Amongst the α-hydroxycarboxylic acids, glycolic acid, lactic acid, citric acid, tartaric acid, malic acid, or mandelic acid are preferred. Pyruvic acid is the preferred α-ketocarboxylic acid.

The aqueous liposome dispersion contains one or more alcohols, preferably at a proportion of up to 20 % by weight. The alcohol is preferably miscible with water and has a preserving effect. It advantageously has a vapour pressure being low enough to enhance penetration of the liposome dispersion into the skin. Monovalent alcohols are preferred. Suitable alcohols are for example ethanol and isopropanol, ethanol being more preferred.

The aqueous liposome dispersion of the invention in a preferred embodiment contains 5 to 20% by weight phospholipids, 5 to 30% by weight α-hydroxycarboxylic acids, α-ketocarboxylic acids or salicylic acid and no more than 20% by weight alcohol. A more preferred composition contains 8 to 12% by weight phospholipids, 15 to 20% by weight acids and no more than 20% by weight alcohol. The said percent values for the acids refer to the total proportion of acids, irrespective of whether the acid molecules are dissociated or undissociated. The dissociated acids are present in their salt form, usually as an alkali salt or alkaline earth salt, the sodium or calcium salts being preferred. The molecular weight of the undissociated acid molecule is used for the calculation of the proportion of acids by weight, irrespective of whether the acid molecules are actually dissociated or undissociated.

In addition to the alcohol, the aqueous liposome dispersion can contain a preservative. Suitable preservatives are, for example, formaldehyde, parabene and Euxyl® K400. If the preservative is present together with alcohol, the alcohol proportion is then usually restricted to 1 to 2% by weight.

The proportion by weight of water in the liposome dispersion is calculated from the proportions of the other components to 100%.

The pH value of the liposome dispersion is in the range from 5 to 7.5. The ideal skin physiological pH is determined by the particular application. A pH in the range from 5.5 to 6.5 is preferred, since generally no skin irritation occurs at these pH values.

For precise setting of the pH value, the aqueous liposome dispersion can contain a buffer system, such as phosphate buffer (KH₂PO₄/Na₂HPO₄).

The pH value set in the liposome dispersion and the acid constants of each α-hydroxycarboxylic and α-ketocarboxylic acid and salicylic acid determine to what extent the acid is present in dissociated form, i.e. as the salt. The pKₐ values for the α-hydroxycarboxylic acids given as examples above all lie within the range from 2.97 to 3.85, only the first dissociation being considered in the case of multivalent carboxylic acids. From these values, using the law of mass action for equilibrium reactions, it can be calculated that the monovalent acids are nearly completely dissociated at the pH value of the invention. For the multivalent acids, this means that at least one proton is dissociated.

The aqueous liposome dispersion can be manufactured, for example, by first dissolving one or more α-hydroxycarboxylic and/or α-ketocarboxylic acids and/or salicylic acid and then adding a base until the required pH is reached. If the carboxylic acid salts are available, these can be dissolved in water and the solution brought to the desired pH by addition of an acid or a base. The salt solution thus obtained is mixed at high energy, for example with a high pressure homogenizer or a slit homogenizer, with a phospholipid mixture which has already been swollen in an aqueous medium to form a lamellar phase. The phospholipids spontaneously form a vesicular lamellar phase under mixing, which encloses the carboxylic acid salts. Further homogenization leads to a uniform distribution of the liposomes (polydispersity < 0.5). The dispersion can subsequently be diluted with alcohol and/or water.

The aqueous liposome dispersions of the present invention have a beneficial effect on the skin, in that the α-hydroxycarboxylic and/or α-ketocarboxylic acids and/or salicylic acid present in their salt form show the keratinolytic and skin stimulating properties described above for the undissociated acids. In some cases, the liposome-enclosed salt forms of the acids have even been observed to be more effective than the undissociated acids usually used.

The liposome dispersions of the invention serve primarily as a base formulation for the manufacture of various liposome preparations for cosmetic and dermatological use. The preferred areas of application are regeneration and moisturization. The liposome dispersions improve the relief of the skin surface, the natural functions of the skin and sloughing, increase the moisture holding capacity and the elasticity of the skin, normalize keratinization and bring about a chemical peeling of the skin.

The weak potency of the acids when not enclosed in liposomes, as in the current state of the art, at higher, non-irritant pH values, i.e. in the weakly acid or neutral range, can be taken to result from the fact that the acids in this pH range are nearly completely present in dissociated form as their salts. The acids in the liposome dispersion of the invention show surprisingly high potency, despite being present at physiological pH as their salts. The assumption that only the undissociated form of the acid is effective must be questioned. Experiments which have been carried out, documented below with lactate as an example, clearly show that the liposome-enclosed acid residue anions possess a considerable skin stimulating effect. The "ineffectiveness" of the acid residue anions when not enclosed in liposomes would appear be due to the fact that the salt ions, in contrast to the undissociated acids, cannot penetrate the skin because of their electrical charge. Enclosure of the salts in liposomes as a carrier system faciliates their penetration into the skin, where the acid residue anion, whose molecular structure is apparently responsible for the mechanism of action, is available to the skin.

The following example of the manufacture of an aqueous liposome dispersion and the results of an experiment will serve to illustrate the present invention and to demonstrate the surprisingly high potency of the liposome dispersion on the skin.

### Example 1: Manufacture of an aqueous liposome dispersion containing lactic acid Composition:

- 29.3% by weight: distilled water
- 20.0% by weight: lactic acid
- 24.0% by weight: sodium hydroxide solution (30%)
- 10.0% by weight: phospholipid fraction, containing
phosphatidyl choline (73-79% by weight)
lysophosphatidyl choline ( 0-6 % by weight)
phosphatidic acid ( < 8 % by weight)
phosphatidyl ethanolamine ( < 4 % by weight)
and other lipids (approx.9 % by weight)
(proportions based on dry weights)
- 16.7% by weight: ethanol, 96%

### Manufacture:

Lactic acid is dissolved in a part of the distilled water and is carefully neutralized with sodium hydroxide solution to a pH of 6.8 to 6.9. The phospholipid fraction is swollen in the reamining distilled water and is added under homogenization using a homogenisator. Subsequently, ethanol is added under stirring. The result is a light yellow, moderately viscous, nearly transparent liposome dispersion.

### Example 2: Comparative study of the effect of lactate enclosed in liposomes in accordance with the present invention, "free" lactate at pH 6.5 and "free" lactic acid at pH 1.8 with respect to skin moisture content and surface structure.

Three samples were tested in comparison with an untreated site (control) on a group of 5 female subjects aged between 28 and 50.

### Sample A:

Aqueous liposome dispersion of the invention consisting of:
10% by weight phospholipids
20% by weight lactic acid
24% by weight sodium hydroxide solution (30%)
16% by weight ethanol
30% by weight distilled water
pH 6.5

### Sample B:

Lactate solution consisting of:
20% by weight lactic acid
24% by weight sodium hydroxide solution (30%)
16% by weight ethanol
40% by weight distilled water
pH 6.5

### Sample C:

Lactic acid solution consisting of:
20% by weight lactic acid
16% by weight ethanol
64% by weight distilled water
pH 6.5

The measurements included changes in the moisture content and surface structure of the skin before and after 8, 21 and 28 days of twice daily application (morning and evening) by the experimental subjects. The inner surface of the forearm was selected as the area to be investigated, and the experimental sites were permanently marked. The measurements were carried out under controlled conditions in a bioclimatized room, a treatment-free period of at least 12 hours being allowed before each measurement (overnight, no morning application).

The measurements of skin moisture content were carried out with the Corneometer CM 820 (Courage and Kazaka). This method is based on simple measurement of the capacitance of the stratum corneum. The underlying principle relies on the very different dielectric constants of water and of other substances.

Profilometry for the determination of skin surface structure was carried out with the analytical instrument Quantimet 970 together with a Polyvar Microscope from Leica/Cambridge Instruments, Bensheim. Video image data transfer from the skin surface takes place via an external macro apparatus. Contact-free imaging of the skin surface from various perspectives is carried out by means of a specially constructed apparatus, the combined data then being evaluated via image analysis. This technique is particularly well established for the analysis of surface structures in the semiconductor industry and in material quality control.

The principle variables of the comparative study were skin moisture content and the surface structure of the skin. The surface structure of the skin was recorded in terms of two measured parameters, skin smoothness and fold count. The variables were evaluated descriptively. The following Figures are based on the means of the measured values.
- Fig. 1:: Skin moisture content
- Fig. 2:: Profilometry/skin smoothness
- Fig. 3:: Profilometry/fold count

### Evaluation of the results

### Skin moisture content:

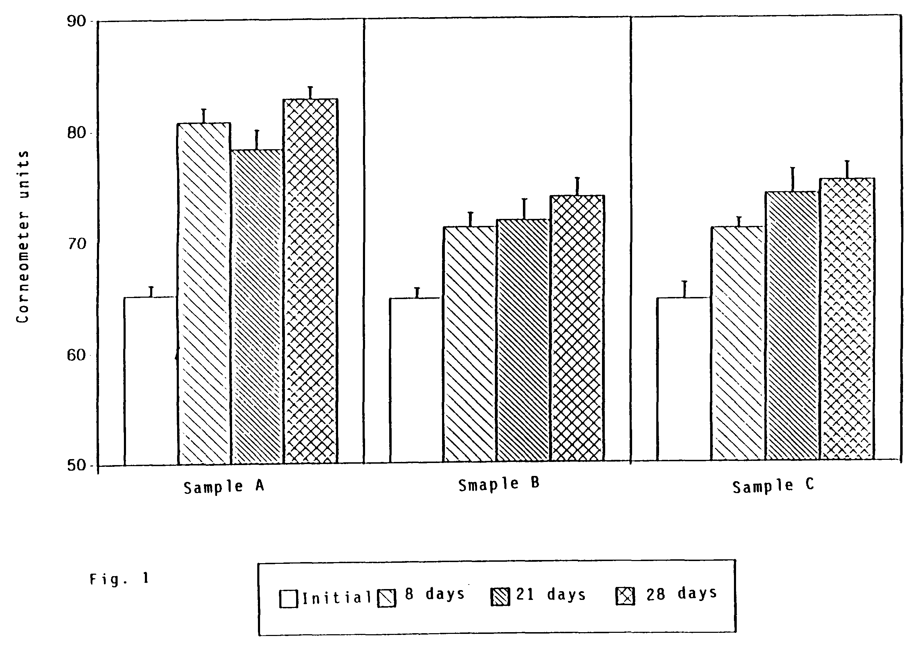

As shown in Fig. 1, the measurements with the corneometer revealed an increase in skin moisture content after only 8 days. No significant further change could be detected after 21 or 28 days. Sample A differed markedly from samples B and C in inducing a more marked increase in skin moisture content. The maximal effect on skin humidity of sample A after four weeks was 29%.

### Contact-free profilometry:

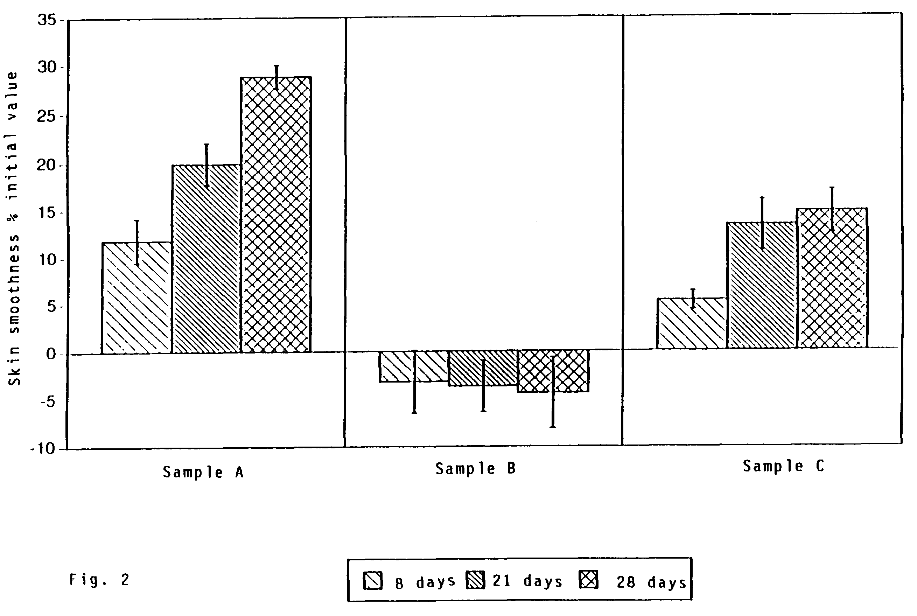

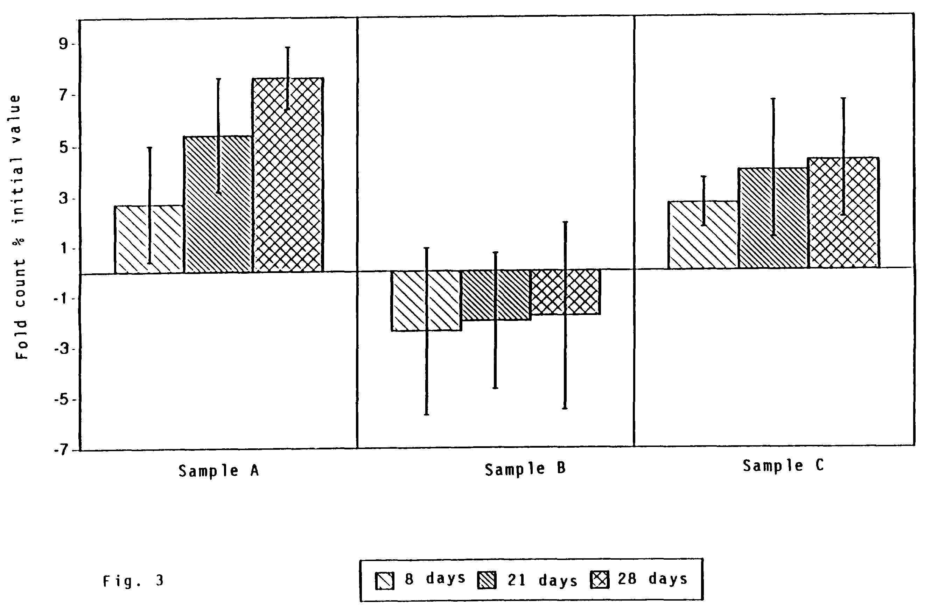

The sites of measurement on the inner side of the forearm were assessed by means of a paired comparison, the same areas being compared before, during and after application. Using a 256-step gray scale, the size and depth of folds were evaluated and were used to calculate the skin smoothness and fold count in terms of % change in the skin surface. Both Fig. 2 and Fig. 3 show clear and contrasting effects of the samples on the surface structure of the skin. Sample A brought about a substantial improvement in skin structure with respect to the depth and number of folds. Sample C also brought about an improvement, although to a much lesser extent. Sample B actually led to a deterioration in skin structure.

The experimental results clearly demonstrate the improved effectiveness of liposome-enclosed lactate compared with lactate not enclosed in liposomes and with lactic acid.

## Claims

1. Aqueous liposomal dispersion of phospholipids comprising:
(a) one or more α-hydroxycarboxylic acids, one or more α-ketocarboxylic acids, or salicylic acid or mixtures therefrom and
(b) one or more alcohols,
**characterized in that**
said dispersion has a physiological pH value in the range from 5 to 7.5 and said acid(s) is (are) present nearly completely in salt form.

2. Aqueous liposomal dispersion of claim 1,
**characterized in that**
the pH value is in the range from 5.5 to 6.5.

3. Aqueous liposomal dispersion of claims 1 or 2,
**characterized in that**
the proportion of said alcohol(s) is no more than 20 % by weight.

4. Aqueous liposomal dispersion of any of claims 1 to 3,
**characterized in that**
the proportion of said phospholipids is 5 to 20 % by weight, the proportion of said acid(s), on the basis of total dissociated und undissociated acid(s), is 5 to 30 % by weight, and the proportion of said alcohol(s) is no more than 20 % by weight.

5. Aqueous liposomal dispersion of any of claims 1 to 4,
**characterized in that**
said phospholipids are obtained from soya and comprise phosphatidyl choline at a proportion of at least 73% by weight.

6. Aqueous liposomal dispersion of any of claims 1 to 4,
**characterized in that**
said phospholipids are hydrogenated and comprise phosphatidyl choline at a proportion of at least 73% by weight.

7. Aqueous liposomal dispersion of any of claims 1 to 6,
**characterized in that**
said α-hydroxycarboxylic acid is selected from glycolic acid, lactic acid, citric acid, tartaric acid, malic acid, and mandelic acid.

8. Aqueous liposomal dispersion of any of claims 1 to 7,
**characterized in that**
said α-ketocarboxylic acid is pyruvic acid.

9. Aqueous liposomal dispersion of any of claims 1 to 8,
**characterized in that**
said alcohol is ethanol or isopropanol.

10. Aqueous liposomal dispersion of any of claims 1 to 9,
**characterized in that**
said liposomes have an average particle size of 70 to 300 nm.

11. Use of the aqueous liposomal dispersion according to any of claims 1 to 10 in cosmetic applications.

12. Use according to claim 11 for regeneration and moisturization of the skin.

## Patentansprüche

1. Wäßrige Liposomendispersion aus Phospholipiden, enthaltend:
(a) eine oder mehrere α-Hydroxycarbonsäuren, eine oder mehrere α-Ketocarbonsäuren oder Salicylsäure oder Mischungen daraus und
(b) einen oder mehrere Alkohole.
dadurch gekennzeichnet, daß die Dispersion einen physiologischen pH-Wert im Bereich von 5 bis 7,5 aufweist und die Säure(n) nahezu vollständig in Form ihrer Salze vorliegt (vorliegen).

2. Wäßrige Liposomendispersion nach Anspruch 1, dadurch gekennzeichnet, daß der pH-Wert im Bereich von 5,5 bis 6,5 liegt.

3. Wäßrige Liposomendispersion nach Anspruch 1 oder 2, dadurch gekennzeichnet, daß der Anteil des Alkohols (der Alkohole) nicht mehr als 20 Gew.-% beträgt.

4. Wäßrige Liposomendispersion nach einem der Ansprüche 1 bis 3, dadurch gekennzeichnet, daß der Anteil der Phospholipide 5 bis 20 Gew.-%, der Anteil der Säure(n), bezogen auf die Gesamtheit aus dissoziierter(n) und undissoziierter(n) Säure(n), 5 bis 30 Gew.-% und der Anteil des Alkohols (der Alkohole) nicht mehr als 20 Gew.-% beträgt.

5. Wäßrige Liposomendispersion nach einem der Ansprüche 1 bis 4, dadurch gekennzeichnet, daß die Phospholipide aus Soja gewonnen sind und einen Phosphatidylcholinanteil von mindestens 73 Gew.-% aufweisen.

6. Wäßrige Liposomendispersion nach einem der Ansprüche 1 bis 4, dadurch gekennzeichnet, daß die Phospholipide hydriert sind und einen Phosphatidylcholinanteil von mindestens 73 Gew.-% aufweisen.

7. Wäßrige Liposomendispersion nach einem der Ansprüche 1 bis 6, dadurch gekennzeichnet, daß die α-Hydroxycarbonsäure aus Glykolsäure, Milchsäure, Citronensäure, Weinsäure, Äpfelsäure und Mandelsäure ausgewählt ist.

8. Wäßrige Liposomendispersion nach einem der Ansprüche 1 bis 7, dadurch gekennzeichnet, daß die α-Ketocarbonsäure Brenztraubensäure ist.

9. Wäßrige Liposomendispersion nach einem der Ansprüche 1 bis 8, dadurch gekennzeichnet, daß der Alkohol Ethanol oder Isopropanol ist.

10. Wäßrige Liposomendispersion nach einem der Ansprüche 1 bis 9, dadurch gekennzeichnet, daß die Liposomen eine mittlere Teilchengröße von 70 bis 300 nm aufweisen.

11. Verwendung der wäßrigen Liposomendispersion nach einem der Ansprüche 1 bis 10 in kosmetischen Anwendungen.

12. Verwendung nach Anspruch 11 zur Regenerierung der Haut und Feuchtigkeitszufuhr für die Haut.

## Revendications

1. Dispersion liposomique aqueuse de phospholipides comprenant :
(a) un ou plusieurs acides α-hydroxycarboxyliques, un ou plusieurs acides α-cétocarboxyliques, ou de l'acide salicylique ou des mélanges de ceux-ci, et
(b) un ou plusieurs alcools,
caractérisée en ce que ladite dispersion a une valeur de pH physiologique dans la gamme de 5 à 7,5 et ledit acide (ou lesdits acides) est (sont) présent(s) presque complètement sous la forme de sel.

2. Dispersion liposomique aqueuse suivant la revendication 1, caractérisée en ce que la valeur de pH se situe dans la gamme de 5,5 à 6,5.

3. Dispersion liposomique aqueuse suivant l'une ou l'autre des revendications 1 et 2, caractérisée en ce que la proportion de l'alcool ou des alcools précités n'est pas supérieure à 20% en poids.

4. Dispersion liposomique aqueuse suivant l'une quelconque des revendications 1 à 3, caractérisée en ce que la proportion desdits phospholipides est de 5 à 20% en poids, la proportion de l'acide ou des acides précités, sur la base de l'acide ou des acides dissociés et non dissociés totaux, est de 5 à 30% en poids, et la proportion de l'alcool ou des alcools précités n'est pas supérieure à 20% en poids.

5. Dispersion liposomique aqueuse suivant l'une quelconque des revendications 1 à 4, caractérisée en ce que les phospholipides sont obtenus à partir de soja et comprennent de la phosphatidyl choline en une proportion d'au moins 73% en poids.

6. Dispersion liposomique aqueuse suivant l'une quelconque des revendications 1 à 4, caractérisée en ce que les phospholipides sont hydrogénés et comprennent de la phosphatidyl choline en une proportion d'au moins 73% en poids.

7. Dispersion liposomique aqueuse suivant l'une quelconque des revendications 1 à 6, caractérisée en ce que l'acide α-hydroxycarboxylique précité est choisi parmi l'acide glycolique, l'acide lactique, l'acide citrique, l'acide tartrique, l'acide malique et l'acide mandélique.

8. Dispersion liposomique aqueuse suivant l'une quelconque des revendications 1 à 7, caractérisée en ce que l'acide α-cétocarboxylique précité est de l'acide pyruvique.

9. Dispersion liposomique aqueuse suivant l'une quelconque des revendications 1 à 8, caractérisée en ce que l'alcool précité est de l'éthanol ou de l'isopropanol.

10. Dispersion liposomique aqueuse suivant l'une quelconque des revendications 1 à 9, caractérisée en ce que lesdits liposomes ont une taille de particule moyenne de 70 à 300 nm.

11. Utilisation de la dispersion liposomique aqueuse suivant l'une quelconque des revendications 1 à 10 dans des applications cosmétiques.

12. Utilisation suivant la revendication 11 pour la régénération et l'hydratation de la peau.
